# FASCICULE DE BREVET EUROPEEN

(11) **EP 0 955 039 B1**
(45) Date de publication et mention de la délivrance du brevet: **19.05.2004**
(21) Numéro de dépôt: 99400598.1
(22) Date de dépôt: 11.03.1999
(51) Int. Cl.: A61K 7/48

(54) **Composition topique contenant un ester d'acide ou d'alcool gras ramifié en C24 à C28**
Topische Zusammensetzung enthaltend einen ester aus einer c24-c28 verzweigte fette Säure oder Alkohol
Topical composition containing an ester of a c24-c28 branched fatty acid or alcohol

(30) Priorité: 31.03.1998 FR 9803979
(43) Date de publication de la demande: 10.11.1999
(73) Titulaire: L'OREAL, 75008 Paris (FR)
(72) Inventeur: Arnaud, Pascal, 94240 L'Hay les Roses (FR); Jacques, Véronique, 92340 Bourg la Reine (FR)
(74) Mandataire: Lhoste, Catherine

(56) Documents cités:
- EP-A- 0 596 284
- EP-A- 0 792 633
- WO-A-93/08840
- WO-A-98/22085
- DE-A- 2 339 149
- DE-A- 19 516 702
- US-A- 5 639 791
- US-A- 5 711 939
- PATENT ABSTRACTS OF JAPAN vol. 017, no. 391 (C & JP 05 070320 A (SHINEI KAGAKU KK)
- PATENT ABSTRACTS OF JAPAN vol. 011, no. 361 (C & JP 62 132807 A (NIPPON OIL & FATS CO.)

## Description

La présente invention se rapporte à une composition topique, cosmétique ou dermatologique contenant un ester de masse moléculaire élevée, présentant des propriétés sensorielles améliorées. Cet ester est un polyester d'acide ou d'alcool gras saturé et ramifié en C₂₄ à C₂₈. L'invention a aussi pour objet l'utilisation de ce polyester dans une composition topique, destinée notamment au soin, traitement ou maquillage de peau aussi bien du visage que du corps humain, des lèvres et des fibres kératiniques ou phanères comme les cheveux, les cils, les sourcils, les ongles.

Il est connu d'utiliser dans la formulation des produits cosmétiques et dermatologiques, des huiles dont le nombre de carbone est supérieur à 50 dans le but d'augmenter par exemple, la brillance d'un rouge à lèvres, la cohésion et l'adhésion d'une poudre, le caractère filmogène et l'émolience d'un produit de soin ou de traitement de la peau.

Dans cette optique le formulateur dispose de plusieurs types de matières premières telles que :
- des polymères huileux, comme les polybutènes qui présentent cependant l'inconvénient d'être très collants,
- des huiles d'origine végétale qui sont le plus souvent oxydables, ce qui peut entraîner une altération des propriétés cosmétiques ou dermatologiques du produit, et qui manifestent aussi un caractère très gras et parfois collant à l'application,
- des esters liquides synthétiques de masses moléculaires élevées, tels que le tétra[isostéarate] de pentaérythrityle (en C₇₇), le citrate de triisoarachidyle (en C₆₆), le tri[isostéarate] de glycéryle (en C₅₇) ou encore le stéaroyl stéarate d'octyldodécyle (en C₅₆) mais qui présentent les inconvénients de manque de glissant à l'application et d'un toucher collant pour les esters de masses moléculaires les plus élevées.

La présente invention a justement pour objet l'utilisation d'un nouvel ester d'acide ou d'alcool gras saturé et ramifié en C₂₄ à C₂₈ dans une composition cosmétique ou pour la préparation d'une composition cosmétique ou dermatologique permettant notamment de remédier à ces inconvénients.

Le demandeur a trouvé de manière inattendue que des esters particuliers, constitués par des acides gras ou alcools gras saturés et ramifiés en C₂₄ à C₂₈, ne présentaient pas les inconvénients cités ci-dessus dans la mesure où, malgré un nombre de carbone élevé, ils possédaient de très bonnes propriétés sensorielles notamment en terme de glissant lors de l'application et d'absence de collant du film déposé sur la peau, les phanères ou les muqueuses telles que les lèvres.

Personne, jusqu'à ce jour n'a décrit ni suggéré d'utiliser ces esters dans une composition à application topique en vue d'apporter, selon l'application spécifique envisagée, de l'émolience, un caractère filmogène, de la brillance, de la cohésion et/ou de l'adhésion à des composés pulvérulents, sans toutefois lui apporter un toucher collant, gras et en améliorant ses propriétés d'étalement et de glissant.

Le résumé de la demande de brevet JP 05 070320 décrit des compositions cosmétiques stables contenant un monoester d'un acide gras saturé en C18 et d'un alcool ramifié, comme le 2-octyldodécanol ou le 2-décyltétradécanol.

Le résumé de la demande de brevet JP 62 132807 décrit une base cosmétique contenant un monoester du propylène glycol et d'un acide gras saturé ramifié en C12-C24. L'acide est par exemple l'acide 2-décyltétradécanoïque.

De façon plus précise l'invention a pour objet une composition topique contenant au moins une phase grasse, caractérisée en ce que cette phase grasse contient au moins un ester d'acide gras ou d'alcool gras saturé et ramifié, la chaîne carbonée de l'acide ou alcool gras étant saturée et ramifiée et contenant 24 à 28 atomes de carbone, l'ester étant un polyester.

L'invention a aussi pour objet l'utilisation d'un polyester d'acide gras ou d'atcool gras dont la chaîne carbonée de l'acide ou de l'alcool est saturée et ramifiée et contient 24 à 28 atomes de carbone, dans une composition cosmétique ou pour la préparation d'une composition dermatologique non collante, non grasse, et/ou brillante lorsqu'elle est appliquée sur la peau, les lèvres et/ou les phanères d'être humain.

L'invention a aussi pour objet l'utilisation d'un polyester d'acide gras ou d'alcool gras dont la chaîne carbonée de l'acide ou de l'alcool est saturée et ramifiée et contient 24 à 28 atomes de carbone, dans une composition cosmétique ou pour la préparation d'une composition dermatologique pour lui conférer un caractère d'émollient et/ou filmogène et/ou de cohésion et d'adhésion lorsque la composition est sous forme pulvérulente.

Le mot ester, selon l'invention, signifie un diester, un triester ou plus généralement un polyester. Il comporte au moins 2 chaînes ramifiées en C₂₄ à C₂₈. Le mot ramifié signifie au moins une chaîne pendante hydrocarbonée comportant notamment de 1 à 14 atomes de carbone.

De préférence, la chaîne pendante comporte au moins 4 atomes de carbone et en particulier 2 carbones de moins que la chaîne principale.

L'ester de l'invention comporte donc un reste d'alcool gras ou acide gras saturé en C₂₄ à C₂₈ notamment du type respectivement alcool gras ou acide gras de Guerbet.

L'ester de la composition de l'invention est, de manière préférentielle, un ester liquide à température ambiante (20-25°C), présentant un poids moléculaire élevé, c'est-à-dire ayant un nombre de carbone supérieur à 50 et notamment supérieur à 70. L'intérêt d'un produit liquide par rapport à un produit pâteux ou solide à température ambiante, réside dans le nombre plus important de ses applications et sa facilité d'utilisation. De plus, le fait que cet ester présente un poids moléculaire élevé permet l'obtention de composition filmogène, rémanente à l'eau, ce qui est largement souhaité pour les produits de protection notamment solaire. Cet ester permet, en outre, l'obtention d'un film brillant, ce qui est recherché par les consommateurs pour certains produits de maquillage comme les vernis à ongles et les rouges à lèvres. Il présente, entre autre, un indice de réfraction supérieur à 1,45 à 20°C et un indice d'iode ≤ 4.

Cet ester malgré sont poids moléculaire élevé, n'est ni gras, ni lourd, ni collant et confère à la composition le contenant des propriétés de confort remarquable. Cet ester est une huile au sens cosmétique du terme et non un tensio-actif.

L'ester selon l'invention est avantageusement un ester huileux d'acide gras ramifié en C₂₄-C₂₈ comme l'acide décyl 2-tétradécanoïque et plus spécialement un ester de polyol comme le glycérol, qui peut être un mono-, di- ou triglycéride. Préférentiellement, cet ester est un triglycéride ramifié en C₂₄-C₂₈ du type de Guerbet, et notamment un triglycéride d'acide en C₂₄ tel que l'acide décyl 2-tétradécanoïque. De préférence, le polyol n'est un ose. Par ailleurs, avantageusement, l'ester est insoluble dans un mélange eau/alcool.

Ce triglycéride est par exemple le tri(décyl 2-tétradécanoate) de glycéryle vendu sous la référence DUB TGI 24 par la société Stéarinerie Dubois. Cet ester présente un indice de saponification de 140 à 150, un indice de réfraction > 1,45 et notamment allant de 1,454 à 1,459, à 20°C, un indice d'iode ≤ 4, un indice d'hydroxyle ≤ 30, un indice d'acide ≤ 10. Son nombre de carbone est de 75.

On peut aussi utiliser les esters d'acide gras en C₂₄ du pentaérythritol comme le tétra(décyl 2-tétradécanoate) de pentaérythrityle (à 101 atomes de carbone) vendu sous la référence DUB PTI 24 par la société Stéarinerie Dubois.

Lorsque l'alcool associé à un acide gras ramifié en C₂₄ à C₂₈ est un polyol, l'estérification peut être partielle (et concerner 1, 2, 3 ou plus de groupes OH selon l'alcool utilisé) ou être totale.

Comme ester de l'invention comportant un reste d'alcool gras à chaîne saturée et ramifiée en C₂₄ à C₂₈, on peut citer les dimérates de di(décyltétradécyle) (à 84 atomes de carbone) comme celui vendu sous la référence DUB DI 24D par la société Stéarinerie Dubois. Les dimérates sont des diacides généralement d'acide en C₆ à C₂₄ comme l'acide oléique, linoleïque, linolénique, etc...

L'ester selon l'invention peut représenter de 0,1 à 99,9 % du poids de la composition, de préférence de 1 à 99 % et mieux de 5 à 90 %, et de façon générale, être présent en une quantité suffisante pour conférer à la composition des propriétés de non gras, non collant, de glissant et/ou de brillance.

Les propriétés sensorielles de l'ester selon l'invention, ont été mises en évidence à travers un test réalisé sur un panel de cinq personnes à partir d'un protocole décrit dans le document EP-A-550779 de la société Nestlé.

Les applications de l'ester de l'invention sont multiples et concernent l'ensemble des produits cosmétiques et dermatologiques.

Les produits de l'invention peuvent se présenter sous la forme de compositions solides, pâteuses ou liquides, anhydres ou en émulsion.

Ainsi, la composition de l'invention peut se présenter sous toutes les formes galéniques normalement utilisées pour une application topique, et notamment sous forme d'un gel huileux, d'une émulsion huile-dans-eau ou eau-dans-huile, d'une dispersion d'huile dans de l'eau grâce à des vésicules, les vésicules étant situées à l'interface huile/eau. Cette composition peut avoir l'aspect d'une crème, d'une pommade, d'une pâte souple, d'un onguent, d'un solide coulé ou moulé et notamment de stick ou de coupelle ou compactée.

De façon plus spécifique, l'invention a pour objet un produit à lèvres contenant une phase grasse liquide contenant au moins un ester tel que défini précédemment.

La composition selon l'invention peut être avantageusement utilisée pour le traitement, le maquillage ou le soin de la peau et/ou des muqueuses selon la nature des actifs utilisés. En particulier, la composition de l'invention peut être un bâton de rouge à lèvres, un brillant à lèvres (gloss en terminologie anglo-saxonne) utilisable tel quel ou pour appliquer sur un film de rouge à lèvres notamment pour en augmenter sa brillance (top coat en terminologie anglo-saxonne). Elle peut aussi constituer un fond de teint fluide ou solide, un produit anti-cernes ou contours des yeux, un eye liner, un mascara, un fard à joues ou à paupières, un vernis à ongles, une poudre libre, un produit de maquillage du corps ou encore un produit de protection solaire ou un produit de soin ou de nettoyage de la peau tel que les produits gommant. Ces compositions peuvent, en outre, contenir des actifs cosmétiques ou dermatologiques, en vue, notamment d'apporter un aspect soin ou traitant à la composition.

Ainsi la composition peut contenir des vitamines et autres actifs lipophiles (lanoline, filtre UVA) ou hydrophiles (hydratants comme la glycérine).

La composition de l'invention peut, en outre, contenir tout autre ingrédient classiquement utilisé dans les domaines concernés. En particulier, elle peut contenir une charge particulaire pouvant représenter de 0 à 35 % du poids total de la composition, de préférence de 0,5 à 20 %, et qui peut comprendre des pigments et/ou des nacres et/ou des charges habituellement utilisés dans les compositions cosmétiques ou dermatologiques. Cette charge peut conduire à une composition colorée, blanche ou incolore.
Par pigments, il faut comprendre des particules blanches ou colorées, minérales ou organiques, insolubles dans la phase grasse liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments peuvent être présents dans la composition à raison de 0,05 à 25 % du poids de la composition finale, et de préférence à raison de 2 à 15 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (DC Red N°7), aluminium.

Les nacres peuvent être présentes dans la composition à raison de 0 à 20 % du poids total de la composition, de préférence à un taux élevé de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 35 % du poids total de la composition, de préférence 0,5 à 15 %. On peut notamment citer le talc, le mica, le kaolin, les poudres de Nylon (Orgasol notamment) et de polyéthylène, le Téflon, l'amidon, le nitrure de bore, des microsphères de copolymères telles que l'Expancel (Nobel Industrie), le polytrap (Dow Corning) et les microbilles de résine de silicone (Tospearl de Toshiba, par exemple).

Avantageusement, la phase grasse contient, de plus, un ou plusieurs autres corps gras et notamment des cires, des gommes et des huiles.

Les cires peuvent être hydrocarbonées, fluorées et/ou siliconées et être d'origine végétale, minérale, animale et/ou synthétique. En particulier, elles présentent une température de fusion supérieure à 25°C et mieux supérieure à 45°C.

Comme cire utilisable dans l'invention, on peut citer, la lanoline oxypropylénée ou non, acétylée ou non, la cire d'abeilles, la cire de Carnauba ou de Candelilla, la paraffine, les cires de lignite ou microcristalline, la cérésine, ou l'ozokérite ; les cires synthétiques comme les cires de polyéthylène et les cires de Fischer-Tropsch ou encore des esters comme l'octacosanylstéarate, les cires de silicones comme les alkyl ou alkoxydiméthicones ayant une chaîne alkyle ou alcoxy de 16 à 45 atomes de carbone.

La nature et la quantité des gommes ou cires sont fonction des propriétés mécaniques et des textures recherchées. A titre indicatif, la composition peut contenir de 0 à 50 % en poids de cires, par rapport au poids total de la composition et mieux de 5 à 30 %.

Les huiles peuvent être des huiles hydrocarbonées et/ou siliconées et/ou fluorées. Ces huiles peuvent être d'origine animale, végétale, minérale ou synthétique. A titre d'exemple d'huile utilisable dans l'invention, on peut citer les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, les triglycérides de noix de coco hydrogénés, et les triglycérides des acides caprylique/caprique comme ceux vendus par la société Stéarineries Dubois ou ceux vendus sous les dénominations Miglyol 810, 812 et 818 par la société Dynamit ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme les huiles de formule R₁COOR₂ dans laquelle R₁ représente le reste d'un acide gras supérieur comportant de 7 à 29 atomes de carbone et R₂ représente une chaîne hydrocarbonée contenant de 3 à 30 atomes de carbone comme par exemple l'huile de Purcellin, le myristate d'isopropyle, le palmitate d'éthyl-2hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles siliconées comme les polydiméthylsiloxanes (PDMS) volatiles ou non, linéaires ou cycliques, liquides ou pâteux à température ambiante ; leurs mélanges.

Ces huiles peuvent représenter de 0 à 99,9 % en poids par rapport à la phase grasse liquide et mieux de 0 à 95 %.

Comme autre ingrédient utilisable dans l'invention, on peut citer les conservateurs, les épaississants de phase aqueuse ou grasse (Bentone ou silice), les parfums, les tensioactifs, les antioxydants et leurs mélanges. Les quantités de ces différents ingrédients sont celles classiquement utilisées dans les domaines concernés et par exemple de 0,01 % à 20 % du poids total de la composition. La nature de ces ingrédients et leur proportion doit être compatibles avec les propriétés recherchées des compositions de l'invention. La composition peut aussi contenir de l'eau à raison de 0 à 95 % du poids total de la composition.

L'invention a encore pour objet une utilisation cosmétique de la composition ci-dessus pour soigner et/ou maquiller la peau et/ou les muqueuses et/ou les phanères et plus spécialement les lèvres ainsi qu'une utilisation de cette composition pour la préparation d'une composition dermatologique destinée à traiter la peau et/ou les muqueuses et notamment les lèvres et/ou les phanères. L'invention a aussi pour objet un procédé de traitement cosmétique et/ou dermatologique de la peau et/ou des muqueuses et notamment des lèvres et/ou des phanères, consistant à appliquer sur la peau et/ou les muqueuses et notamment les lèvres et/ou les phanères d'être humain la composition définie ci-dessus.

L'invention a encore pour objet un procédé pour conférer à une composition topique un toucher non collant et/ou non gras et/ou un aspect brillant, et/ou un caractère filmogène consistant à introduire dans un milieu cosmétique et/ou dermatologique, au moins un ester tel que défini précédemment. Elle a encore pour objet un procédé pour conférer à une composition topique pulvérulente de la cohésion et/ou de l'adhésion, consistant à introduire dans la composition au moins un ester tel que défini précédemment.

La composition de l'invention peut être obtenue par chauffage des différents constituants à la température de fusion des cires la plus élevée, puis coulage du mélange fondu dans un moule (coupelle ou doigt de gant). Elle peut aussi être obtenue par extrusion comme décrit dans la demande EP-A-667 146.

Les exemples de compositions ci-après sont donnés à titre illustratif et sans caractère limitatif. Les quantités y sont données en % en poids.

### Exemple 1: gloss en bouillotte

| | |
|---|---|
| Triglycéride en C₂₄ (DUB TGI 24) | 81 % |
| Silice pyrogénée hydrophobe (Aérosil R972 | 9 % |
| de Degussa) | |
| Nacres | 9,5 % |
| Conservateur, antioxydant | 0,5 % |

Mode opératoire : On disperse la silice dans l'huile à température ambiante à l'aide d'un Rayneri à une vitesse de 1 000 tr/min. On ajoute les nacres en maintenant l'agitation et on verse la composition dans des bouillottes. On obtient ainsi un gloss très brillant facile à appliquer et très peu collant.

### Exemple 2 : gloss en pot

| | |
|---|---|
| Triglycéride en C₂₄ (DUB TGI 24) | 34,0 % |
| Huile de lanoline | 40,0 % |
| Octyldodécanol | 14,0 % |
| Cire de polyéthylène | 6,0 % |
| Cire microcristalline | 3,0 % |
| Pigment | 2,0 % |
| Conservateur, antioxydant | 0,5 % |
| Parfum | 0,5 % |

Mode opératoire : Les constituants sont mélangés et chauffés à 100-105°C. Après homogénéisation et broyage des pigments, le mélange est coulé à 100°C dans des pots appropriés. On obtient ainsi un gloss très brillant et peu collant.

### Exemple 3 : rouge à lèvres en stick

| | |
|---|---|
| Triglycéride en C₂₄ (DUB TGI 24) | 7,0 % |
| Dimèredilinoléate de dioctyldodécyle | 24,0 % |
| Octyldodécanol | 15,0 % |
| Triglycéride d'acide caprique/caprylique | 10,0 % |
| Bentone (quaternium-18 hectorite) | 0,6 % |
| Huile de lanoline | 11,0 % |
| Lanoline oxypropylénée (5 OP) | 5,0 % |
| Lanoline acétylée | 5,0 % |
| Cire de polyéthylène | 8,0 % |
| Octacosanylstéarate | 5,0 % |
| Glycérides de noix de coco hydrogénés | 2,0 % |
| Pigments | 7,0 % |
| Parfum, conservateur, antioxydant | 0,4 % |

Mode opératoire : On disperse la bentone dans une partie de la phase huileuse, puis on ajoute le reste de la phase grasse que l'on chauffe à 95°C. Après homogénéisation et broyage des pigments, le mélange est coulé dans des moules adéquates. On obtient alors un stick de rouge à lèvres présentant de bonnes propriétés cosmétiques d'application et de brillance.

### 1er) Test comparatif

### a) Test

Le test consiste en une comparaison, à travers une formule simple sous forme de gel de silice, des propriétés cosmétiques de l'ester de l'invention (ici le DUB TGI 24) (composition B) avec celles du tétra[isostéarate] de pentaérithrityle (composition A) et du tri[isostéarate] de glycéryle (composition C).

Le tétra[isostéarate] de pentaérythrityle (en C₇₇) a été choisi comme représentant un ester huileux de masse moléculaire élevée. Le tri[isostéarate] de glycéryle (en C₅₇) a été choisi comme représentant un ester ayant une structure chimique proche de celle de l'ester de l'invention.

La comparaison a été réalisée à partir des formules suivantes :

| **COMPOSITIONS** | **A** | **B** | **C** |
|---|---|---|---|
| • Silice pyrogénée vendue sous la réf. Aérosil R972 par Degussa | 9,00 % | 9,00 % | 9,00 % |
| • Tétra[isostéarate] de Pentaérythrityle vendu sous la référence Prisorine PTIS 3631 de Unichema | 90,13 % | - | - |
| • Ester de l'invention vendu sous la référence DUB TGI 24 par Stéarinerie Dubois | - | 90,13 % | - |
| • Tri[isostéarate] de glycéryle vendu par Stéarinerie Dubois | - | - | 90,13 % |
| • Conservateur | 0,40 % | 0,40 % | 0,40 % |
| • Antioxydant | 0,07 % | 0,07 % | 0,07 % |
| • Parfum | 0,40 % | 0,40 % | 0,40 % |

Pour chaque formule la silice a été dispersée dans l'huile au Rayneri à température ambiante.

### b) Protocole du test

Le test a été réalisé sur 5 personnes en suivant le protocole décrit dans le document EP 550 779 de Nestlé. Cette méthode permet en effet de quantifier numériquement par un indice (IDT, indice de toucher) les différentes caractéristiques qui définissent le toucher des produits cosmétiques.

L'IDT comprend trois paramètres :
- Le toucher initial comprenant :
   - une note de texture de 1 (trop sèche) à 5 (très grasse),
   - une note de glissement de 1 (freine trop) à 5 (glisse bien),
   la cotation constituant la moyenne des deux notes.
- Le toucher intermédiaire pendant l'étalement comprenant :
   - un note d'étalement de 1 (freine trop) à 5 (glisse bien),
   - une note de caractère collant de 1 (trop collant) à 5 (pas collant),
   - une note de vitesse de pénétration de 1 (très lente) à 5 (rapide),
   - une note de degré de pénétration de 1 (inexistante) à 5 (très bonne),
   la cotation constituant la moyenne des quatre notes.
- Le toucher final comprenant :
   - une note d'impression au frottement de 1 (freine trop) à 5 (glisse bien),
   - une note caractérisant le film lipidique résiduel sur la peau de 1 (inexistant, peau sèche) à 5 (riche, peau bien nourrie),
   la cotation étant la somme des deux notes.

L'IDT est exprimé par la cotation du toucher initial/la somme de la cotation du toucher intermédiaire et de la cotation du toucher final. Les échantillons de produits à tester sont soumis aux expérimentateurs qui les appliquent successivement sur les intérieurs des avants-bras. La quantité de produit à appliquer doit être la même pour chaque essai, c'est-à-dire environ 0,2 g.

### c) Résultats du test

**Tableau I**

| **COMPOSITIONS** | **A** | **B** | **C** |
|---|---|---|---|
| **Toucher initial** | | | |
| Note de texture | 3,60 | 3,40 | 3,70 |
| Note de glissement | 2,90 | 4,20 | 3,40 |
| Moyenne | 3,25 | 3,80 | 3,55 |

| **Toucher intermédiaire** | | | |
|---|---|---|---|
| Note d'étalement | 2,00 | 3,90 | 3,40 |
| Note de caractère collant | 1,50 | 3,50 | 3,20 |
| Note de vitesse de pénétration | 2,80 | 2,50 | 1,30 |
| Note de degré de pénétration | 2,60 | 2,30 | 2,00 |
| Moyenne | 2,23 | 3,05 | 2,48 |

| **Toucher Final** | | | |
|---|---|---|---|
| Note de frottement | 2,30 | 4,10 | 3,40 |
| Note de film lipidique résiduel | 3,00 | 3,70 | 2,80 |
| Somme | 5,30 | 7,80 | 6,20 |
| **IDT** | 3,25/7,53 | 3,80/10,85 | 3,55/8,68 |

Les propriétés sensorielles sont d'autant meilleures que le numérateur et le dénominateur de l'IDT sont élevés.

Il ressort clairement de ce test que l'ester selon l'invention procure des propriétés améliorées par rapport à ceux de l'art antérieur.

Bien entendu, les propriétés cosmétiques de ce triglycéride d'acide gras ramifié en C₂₄ sont tout à fait comparables à celles des esters d'alcool gras ramifiés en C₂₄ et comportant un nombre d'atomes total similaire.

### 2ème) Test comparatif

Dans ce test, on a comparé les propriétés cosmétiques de l'ester de l'invention (ici le DUB PTI 24), composition E avec celles du tétra[isostéarate] de pentaérythrityle (à 77 atomes de carbone), vendu sous la référence Prisorine (PTIS 3631) par la société Unichema, composition D.

La comparaison a été effectuée dans les mêmes conditions qu'au point 1) sur les compositions D et E suivantes. Les résultats de ce test sont portés dans le tableau II.

| **COMPOSITIONS** | **D** | **E** |
|---|---|---|
| Silice pyrogénée vendue sous la réf. Aérosil R972 par Degussa | 9,00 % | 9,00 % |
| Tétra[isostéarate] de Pentaérythrityle vendu sous la forme PTIS 3631 | 90,13 % | |
| Tétra[décyltétradécanoate] de pentaérythytyle (DUB PTI24) | | 90,13 % |
| Conservateur | 0,40 % | 0,40 % |
| Antioxydant | 0,07 % | 0,07 % |
| Parfum | 0,40 % | 0,40 % |

**Tableau II**

| **COMPOSITIONS** | **D** | **E** |
|---|---|---|
| Toucher initial | | |
| Note de texture | 4,00 | 4,00 |
| Note de glissement | 2,60 | 2,60 |
| Moyenne | 3,30 | 3,30 |

| Toucher intermédiaire | | |
|---|---|---|
| Note d'étalement | 2,40 | 2,80 |
| Note de caractère collant | 2,80 | 3,40 |
| Note de vitesse de pénétration | 2,40 | 2,00 |
| Note de degré de pénétration | 2,80 | 2,20 |
| Moyenne | 2,60 | 2,60 |

| Toucher final | | |
|---|---|---|
| Note de frottement | 2,20 | 2,80 |
| Note de film lipidique résiduel | 3,20 | 3,80 |
| Somme | 5,40 | 6,60 |
| IDT | 3,30/8,00 | 3,30/9,20 |

II ressort clairement du tableau Il que l'ester de l'invention procure des propriétés améliorées par rapport à celles de l'ester ne comportant pas de chaîne ramifiée en C₂₄-C₂₈.

### 3ème) Test comparatif

Dans ce test, on a comparé les propriétés cosmétiques de l'ester selon l'invention (ici le DUB DI 24 D), composition G, avec celles d'un dimère de l'art antérieur, le dilinoléate de di(octyldodécyle) (à 76 atomes de carbone) vendu sous la référence Liquiwax DIEFA par la société Brooks Ind., composition F. Les compositions F et G sont données ci-après et les résultats du tests sont portés dans le tableau III.

| **COMPOSITIONS** | **F** | **G** |
|---|---|---|
| Silice pyrogénée vendue sous la réf. Aérosil R972 par Degussa | 9,00 % | 9,00 % |
| Dimère dilinoleate de di(octyldocécyle) (Liquiwax DIEFA) | 90,13 % | |
| Di(décyltétradécyl) dimérate (DUB DI 24 D) | | 90,13 % |
| Conservateur | 0,40 % | 0,40 % |
| Antioxydant | 0,07 % | 0,07 % |
| Parfum | 0,40 % | 0,40 % |

**Tableau III**

| **COMPOSITIONS** | **F** | **G** |
|---|---|---|
| Toucher initial | | |
| Note de texture | 4,00 | 3,80 |
| Note de glissement | 3,00 | 4,00 |
| Moyenne | 3,50 | 3,90 |

| Toucher intermédiaire | | |
|---|---|---|
| Note d'étalement | 2,70 | 3,60 |
| Note de caractère collant | 2,60 | 3,40 |
| Note de vitesse de pénétration | 2,00 | 2,60 |
| Note de degré de pénétration | 2,60 | 2,80 |
| Moyenne | 2,50 | 3,10 |

| Toucher final | | |
|---|---|---|
| Note de frottement | 2,00 | 3,40 |
| Note de film lipidique résiduel | 3,60 | 3,60 |
| Somme | 5,60 | 7,00 |
| IDT | 3,50/8,10 | 3,90/10,10 |

Là encore, l'ester de l'invention (reste d'alcool gras en C₂₄) procure des propriétés améliorées par rapport à celles de l'ester de l'art antérieur (sans chaîne ramifiée en C₂₄ à C₂₈).

## Revendications

1. Composition topique contenant au moins une phase grasse, **caractérisée en ce que** cette phase grasse contient au moins un ester d'acide gras ou d'alcool gras, la chaîne carbonée de l'acide ou alcool gras étant saturée et ramifiée et contenant 24 à 28 atomes de carbone et l'ester étant un polyester.

2. Composition selon la revendication 1, **caractérisée en ce que** l'ester comporte un nombre de carbone > 50 et mieux > 70.

3. Composition selon l'une des revendications précédentes, **caractérisée en ce que** l'ester est un ester d'acide ou d'alcool gras en C₂₄.

4. Composition selon l'une des revendications précédentes, **caractérisée en ce que** l'ester est liquide à température ambiante.

5. Composition selon l'une des revendications précédentes, **caractérisée en ce que** l'ester est choisi parmi les triglycérides d'acide gras ramifié en C₂₄, les esters d'acide gras ramifié en C₂₄ du pentaérythritol, les esters d'alcool gras ramifié en C₂₄ et de diacides, et leurs mélanges.

6. Composition selon l'une des revendications précédentes, **caractérisée en ce que** l'indice de réfraction de l'ester est supérieur à 1,45 à 20°C.

7. Composition selon l'une des revendications précédentes, **caractérisée en ce que** l'ester présente un indice d'iode ≤ 4.

8. Composition selon l'une des revendications précédentes, **caractérisée en ce que** l'ester est un triglycéride d'acide gras ramifié en C₂₄.

9. Composition selon l'une des revendications précédentes, **caractérisée en ce que** l'ester est le triglycéride de l'acide décyl-2-tétradécanoïque.

10. Composition selon l'une des revendications précédentes, **caractérisée en ce que** l'ester est en quantité suffisante pour conférer à la composition des propriétés de non gras, de non collant, de glissant et/ou de brillance.

11. Composition selon l'une des revendications précédentes, se présentant sous forme de produit de maquillage et/ou de soin du visage ou du corps, des lèvres et/ou des phanères.

12. Composition de soin ou de maquillage des lèvres, **caractérisée en ce qu'**elle est conforme à l'une des revendications précédentes.

13. Composition selon l'une des revendications précédentes, **caractérisée en ce qu'**elle contient, entre autre, au moins une cire et/ou au moins une charge particulaire et/ou au moins une huile différente dudit ester.

14. Utilisation d'un polyester saturé d'acide gras ou d'alcool gras dont la chaîne carbonée de l'acide ou de l'alcool est ramifiée et contient 24 à 28 atomes de carbone, dans une composition cosmétique ou pour la préparation d'une composition dermatologique non collante, non grasse, et/ou brillante lorsqu'elle appliquée sur la peau, les lèvres et/ou les phanères d'être humain.

15. Utilisation d'un polyester saturé d'acide gras ou d'alcool gras dont la chaîne carbonée de l'acide ou de l'alcool est ramifiée et contient 24 à 28 atomes de carbone, dans une composition cosmétique ou pour la préparation d'une composition dermatologique, en vue de lui conférer un caractère émollient et/ou filmogène et/ou de cohésion et d'adhésion lorsque la composition est sous forme pulvérulente.

16. Utilisation selon la revendication 14 ou 15, **caractérisée en ce que** l'ester est liquide à température ambiante.

17. Utilisation selon l'une des revendications 14 à 16, **caractérisée en ce que** l'ester est choisi parmi les triglycérides d'acide gras ramifié en C₂₄, les esters d'acide gras ramifié en C₂₄ du pentaérythrytol, les esters d'alcool gras ramifié en C₂₄ et de diacides, et leurs mélanges.

18. Utilisation selon l'une des revendications 14 à 17, **caractérisée en ce que** l'ester est un triglycéride d'acide gras ramifié en C₂₄.

19. Utilisation selon d'une des revendications 14 à 18, **caractérisée en ce que** l'ester est le triglycéride de l'acide décyl 2-tétradécanoïque.

20. Procédé de traitement cosmétique de la peau et/ou des muqueuses et notamment des lèvres et/ou des phanères, consistant à appliquer sur la peau et/ou les muqueuses et notamment les lèvres et/ou les phanères d'être humain, la composition selon l'une des revendications 1 à 13.

21. Procédé pour conférer à une composition topique un toucher non collant et/ou non gras et/ou un aspect brillant, et/ou un caractère filmogène, consistant à introduire dans un milieu cosmétique et/ou dermatologique au moins un ester tel que défini dans les revendications 1 à 9.

22. Procédé pour conférer à une composition topique pulvérulente de la cohésion et/ou de l'adhésion, consistant à introduire dans la composition au moins un ester tel que défini dans les revendications 1 à 9.

## Patentansprüche

1. Zusammensetzung zur topischen Anwendung, die mindestens eine Fettphase enthält, **dadurch gekennzeichnet, dass** die Fettphase mindestens einen Ester einer Fettsäure oder eines Fettalkohols enthält, wobei die Kohlenstoffkette der Fettsäure oder des Fettalkohols gesättigt und verzweigt ist und 24 bis 28 Kohlenstoffatome enthält und wobei der Ester ein Polyester ist.

2. Zusammensetzung nach Anspruch 1, **dadurch gekennzeichnet, dass** der Ester > 50 und besser > 70 Kohlenstoffatome enthält.

3. Zusammensetzung nach einem der vorhergehenden Ansprüche, **dadurch gekennzeichnet, dass** der Ester ein Ester einer Fettsäure oder eines Fettalkohols mit 24 Kohlenstoffatomen ist.

4. Zusammensetzung nach einem der vorhergehenden Ansprüche, **dadurch gekennzeichnet, dass** der Ester bei Raumtemperatur flüssig ist.

5. Zusammensetzung nach einem der vorhergehenden Ansprüche, **dadurch gekennzeichnet, dass** der Ester unter den Triglyceriden einer verzweigten C₂₄-Fettsäure, den Pentaerythritestern einer verzweigten C₂₄-Fettsäure, den Estern von verzweigten C₂₄-Fettalkoholen und Disäuren und deren Gemischen ausgewählt ist.

6. Zusammensetzung nach einem der vorhergehenden Ansprüche, **dadurch gekennzeichnet, dass** der Ester bei 20 °C einen Brechungsindex über 1,45 aufweist.

7. Zusammensetzung nach einem der vorhergehenden Ansprüche, **dadurch gekennzeichnet, dass** der Ester eine Iodzahl ≤ 4 aufweist.

8. Zusammensetzung nach einem der vorhergehenden Ansprüche, **dadurch gekennzeichnet, dass** der Ester ein Triglycerid einer verzweigten C₂₄-Fettsäure ist.

9. Zusammensetzung nach einem der vorhergehenden Ansprüche, **dadurch gekennzeichnet, dass** es sich bei dem Ester um das Triglycerid der 2-Decyltetradecansäure handelt.

10. Zusammensetzung nach einem der vorhergehenden Ansprüche, **dadurch gekennzeichnet, dass** der Ester in einer Menge enthalten ist, die ausreichend ist, damit sich die Zusammensetzung nicht klebrig und/oder nicht fettig anfühlt, und/oder damit sie gleitet und/oder um ihr ein glänzendes Aussehen zu geben.

11. Zusammensetzung nach einem der vorhergehenden Ansprüche, die als Produkt zum Schminken und/oder zur Pflege des Gesichts oder des Körpers, der Lippen und/oder der Hautanhangsgebilde vorliegt.

12. Zusammensetzung zur Pflege und/oder zum Schminken der Lippen, **dadurch gekennzeichnet, dass** sie einem der vorhergehenden Ansprüche entspricht.

13. Zusammensetzung nach einem der vorhergehenden Ansprüche, **dadurch gekennzeichnet, dass** sie ferner mindestens ein Wachs und/oder mindestens eine Füllstoff in Partikelform und/oder mindestens ein von dem Ester verschiedenes Öl enthält.

14. Verwendung eines gesättigten Polyesters einer Fettsäure oder eines Fettalkohols, wobei die Kohlenstoffkette der Fettsäure oder des Fettalkohols verzweigt ist und 24 bis 28 Kohlenstoffatome enthält, in einer kosmetischen Zusammensetzung oder zur Herstellung einer dermatologischen Zusammensetzung, die nach dem Auftragen auf die menschliche Haut, die Lippen und/ oder die Hautanhangsgebilde nicht klebrig sind, nicht fettig sind und/oder glänzen.

15. Verwendung eines gesättigten Polyesters einer Fettsäure oder eines Fettalkohols, wobei die Kohlenstoffkette der Fettsäure oder des Fettalkohols verzweigt ist und 24 bis 28 Kohlenstoffatome enthält, in einer kosmetischen Zusammensetzung oder zur Herstellung einer dermatologischen Zusammensetzung, um ihnen weich machende Eigenschaften und/oder einen filmbildenden Charakter und/oder, wenn die Zusammensetzung in Pulverform vorliegt, Kohäsion und/ oder Adhäsion zu geben.

16. Verwendung nach Anspruch 14 oder 15, **dadurch gekennzeichnet, dass** der Ester bei Raumtemperatur flüssig ist.

17. Verwendung nach einem der Ansprüche 14 bis 16, **dadurch gekennzeichnet, dass** der Ester unter den Triglyceriden einer verzweigten C₂₄-Fettsäure, den Pentaerythritestern einer verzweigten C₂₄-Fettsäure, den Estern von verzweigten C₂₄-Fettalkoholen und Disäuren und deren Gemischen ausgewählt ist.

18. Verwendung nach einem der Ansprüche 14 bis 17, **dadurch gekennzeichnet, dass** der Ester ein Triglycerid einer verzweigten C₂₄-Fettsäure ist.

19. Verwendung nach einem der Ansprüche 14 bis 18, **dadurch gekennzeichnet, dass** es sich bei dem Ester um das Triglycerid der 2-Decyltetradecansäure handelt.

20. Verfahren zur kosmetischen Behandlung der Haut und/oder der Schleimhäute und insbesondere der Lippen und/oder der Hautanhangsgebilde, das darin besteht, auf die menschliche Haut und/oder die Schleimhäute und insbesondere die Lippen und/ oder die Hautanhangsgebilde eine Zusammensetzung nach einem der Ansprüche 1 bis 12 aufzubringen.

21. Verfahren, das dazu dient, dass sich eine Zusammensetzung zur topischen Anwendung nicht klebrig und/oder nicht fettig anfühlt und/oder um ihr ein glänzendes Aussehen und/oder einen filmbildenden Charakter zu geben und das darin besteht, in ein kosmetisch und/oder dermatologisch akzeptables Medium mindestens einen Ester nach einem der Ansprüche 1 bis 9 einzuarbeiten.

22. Verfahren, das dazu dient, einer in Pulverform vorliegenden Zusammensetzung zur topischen Anwendung Kohäsion und/oder Adhäsion zu geben und das darin besteht, in die Zusammensetzung mindestens einen Ester nach einem der Ansprüche 1 bis 9 einzuarbeiten.

## Claims

1. Topical composition comprising at least one fatty phase, **characterized in that** this fatty phase comprises at least one fatty alcohol or fatty acid ester, the carbon-comprising chain of the fatty alcohol or acid being saturated and branched and comprising 24 to 28 carbon atoms and the ester being a polyester.

2. Composition according to Claim 1, **characterized in that** the ester comprises a carbon number > 50 and better still > 70.

3. Composition according to either of the preceding claims, **characterized in that** the ester is a C₂₄ fatty alcohol or acid ester.

4. Composition according to one of the preceding claims, **characterized in that** the ester is liquid at room temperature.

5. Composition according to one of the preceding claims, **characterized in that** the ester is chosen from branched C₂₄ fatty acid triglycerides, branched C₂₄ fatty acid esters of pentaerythritol, branched C₂₄ fatty alcohol esters of diacids, and their mixtures.

6. Composition according to one of the preceding claims, **characterized in that** the refractive index of the ester is greater than 1.45 at 20°C.

7. Composition according to one of the preceding claims, **characterized in that** the ester exhibits an iodine number ≤ 4.

8. Composition according to one of the preceding claims, **characterized in that** the ester is a branched C₂₄ fatty acid triglyceride.

9. Composition according to one of the preceding claims, **characterized in that** the ester is the triglyceride of 2-decyltetradecanoic acid.

10. Composition according to one of the preceding claims, **characterized in that** the ester is in an amount sufficient to confer non-greasy, non-sticky, slip and/or gloss properties on the composition.

11. Composition according to one of the preceding claims, which is provided in the form of a product for making up and/or caring for the face or body, lips and/or superficial body growths.

12. Composition for caring for or making up the lips, **characterized in that** it is in accordance with one of the preceding claims.

13. Composition according to one of the preceding claims, **characterized in that** it comprises, inter alia, at least one wax and/or at least one particulate filler and/or at least one oil other than the said ester.

14. Use of a fatty alcohol or fatty acid saturated polyester, the carbon-comprising chain of the acid or of the alcohol of which is branched and comprises 24 to 28 carbon atoms, in a cosmetic composition or for the preparation of a dermatological composition which is non-sticky, non-greasy and/or glossy when it is applied to the skin, lips and/or superficial body growths of a human being.

15. Use of a fatty alcohol or fatty acid saturated polyester, the carbon-comprising chain of the acid or of the alcohol of which is branched and comprises 24 to 28 carbon atoms, in a cosmetic composition or for the preparation of a dermatological composition for the purpose of conferring on it an emollient and/or film-forming and/or cohesive and adhesive nature when the composition is in pulverulent form.

16. Use according to Claim 14 or 15, **characterized in that** the ester is liquid at room temperature.

17. Use according to one of Claims 14 to 16, **characterized in that** the ester is chosen from branched C₂₄ fatty acid triglycerides, branched C₂₄ fatty acid esters of pentaerythritol, branched C₂₄ fatty alcohol esters of diacids, and their mixtures.

18. Use according to one of Claims 14 to 17, **characterized in that** the ester is a branched C₂₄ fatty acid triglyceride.

19. Use according to one of Claims 14 to 18, **characterized in that** the ester is the triglyceride of 2-decyltetradecanoic acid.

20. Process for the cosmetic treatment of the skin and/or mucous membranes and in particular of the lips and/or superficial body growths which consists in applying the composition according to one of Claims 1 to 13 to the skin and/or mucous membranes and in particular the lips and/or superficial body growths of human beings.

21. Process for conferring a non-sticky and/or non-greasy feel and/or a glossy appearance and/or a film-forming nature on a topical composition which consists in introducing at least one ester as defined in Claims 1 to 9 into a cosmetic and/or dermatological medium.

22. Process for conferring cohesion and/or adhesion on a pulverulent topical composition which consists in introducing at least one ester as defined in Claims 1 to 9 into the composition.
